# EUROPEAN PATENT SPECIFICATION

(11) **EP 4 238 436 B1**
(45) Date of publication and mention of the grant of the patent: **22.04.2026**
(21) Application number: 20959973.7
(22) Date of filing: 29.10.2020
(51) Int. Cl.: A41C 3/00, A41C 3/08, A61F 5/02, A61F 5/03

(54) **WOMEN'S TOP FOR BREAST SHAPING AND THORACIC SPINE CORRECTION**
FRAUENOBERTEIL ZUR BRUSTUMFORMUNG UND KORREKTUR DER BRUSTWIRBELSÄULE
HAUT DE FEMME POUR MISE EN FORME DE POITRINE ET CORRECTION DE COLONNE THORACIQUE

(43) Date of publication of application: 06.09.2023
(73) Proprietor: Slimminggo Group, Jeju-do 63141 (KR)
(72) Inventor: EOM, Tae Ho, Seoul 05693 (KR); LEE, Seung Ah, Seoul 05578 (KR)
(74) Representative: Groth & Co. KB
(86) International application number: PCT/KR2020/014935
(87) International publication number: WO 2022/092356

(56) References cited:
- CN-A- 110 623 321
- JP-A- 2007 138 335
- JP-A- 2013 085 696
- JP-U- 3 115 670
- KR-A- 20190 041 021
- KR-U- 20130 004 794
- US-A1- 2012 059 297
- US-A1- 2015 094 634
- US-A1- 2015 094 634
- US-A1- 2016 278 963
- US-A1- 2017 354 530
- US-A1- 2019 029 866

## Description

### [Technical Field]

The present embodiment relates to a women's top for breast shaping and thoracic spine correction.

### [Background Art]

In general, women's brassiere underwear is clothing worn on the inside the garment, and other functions other than the use of clothing or beauty cannot be expected. In particular, the effect of correcting the backbone or spine could not be expected.

Modern people are engaged in various jobs, and most of their tasks are to organize or process documents for a long time with their shoulders bent in front of their desks, so if they do these tasks for a long time, problems such as bending their upper body forward and bending their spine can occur.

In order to prevent bending of the spine due to forward bending of the upper body, it is necessary to always maintain a correct posture while working and exercise regularly, but it is difficult for busy modern people.

Therefore, various types of compression clothing, correction underwear, and correction devices are being developed to prevent the upper body from bending forward, but there is a problem in that satisfactory effects are not obtained.

US2012059297 Al discloses the preamble of claim 1.

### [Detailed Description of the Invention]

### [Technical Subject]

The problem that the present embodiment is trying to solve is to provide a women's top for upper body correction that supports the thoracic spine and straightens the back while at the same time pulling the breast in the opposite direction of gravity and enhancing the volume with a beautiful triangular line which is a differentiated female body characteristic from men.

### [Technical Solution]

The invention is set out in the appended set of claims.

A women's top for breast shaping and thoracic spine correction according to the present invention comprises: a main body covering the upper body of a user; a first band being disposed on one side of the main body; a second band being disposed on the other side of the main body and at least a part of which is connected, on the front of the main body, to the first band; and a lumbar vertebra support being disposed on the back of the main body, wherein the lumbar vertebra support includes a first section being overlapped with the first band, a second section being overlapped with the second band, and a third section being extended downward from the first and second sections.

In addition, each of the first and second bands includes first and second round parts being disposed on the front surface of the main body, and one end portion of the first round part is coupled with one end portion of the second round part.

In addition, the first band includes a first portion being extended upward from the other end portion of the first round part, and the second band includes a first portion being extended upward from the other end portion of the second round part.

In addition, the first band includes a second portion being extended from the first portion of the first band to the rear surface of the main body, the second band includes a second portion being extended from the first portion of the second band to the rear surface of the body, and the second portion of the first band and the second portion of the second band are intersected at the rear surface of the main body.

In addition, the first band includes a third portion being extended from the second portion of the first band to the front surface of the main body; the second band includes a third portion being extended from the second portion of the second band to the front surface of the body; the third portion of the first band and the third portion of the second band are disposed below the first round part and the second round part; and the third portion of the first band and the third portion of the second band are coupled to each other and fixed to the main body.

In addition, the third portion of the first band is pulled in a first direction, and the third portion of the second band is pulled in a second direction being intersected with the first direction.

In addition, when the third portion of the first band is pulled in the first direction and the third portion of the second band is pulled in the second direction, the first round part and the second round part are pulled in a third direction.

In addition, it may be greater than the degree to which the third region of the lumbar vertebra support is deformed.

In addition, the maximum width in a horizontal direction between the first section and the second section of the lumbar vertebra support may be greater than the width of the third section of the lumbar vertebra support in a corresponding direction.

In addition, the shortest width in the horizontal direction between the first section and the second section of the lumbar vertebra support may be greater than a width of the third section of the lumbar vertebra support in a corresponding direction.

### [Advantageous Effects]

Through present embodiment, it is possible to provide a female top for upper body correction that supports the thoracic vertebrae and straightens the back while pulling the breast in the opposite direction of gravity and enhancing the volume with a beautiful triangular line, which is a characteristic of the female body differentiated from that of men,.

### [Brief Description of Drawings]

FIG. 1 is a front view of a female top for breast correction and thoracic spine correction according to present invention.
FIG. 2 is a rear view of a female top for breast correction and thoracic spine correction according to the present invention.
FIG. 3 is a rear view of a female top for breast correction and thoracic spine correction according to the present invention.

### [Specific Contents for Implementing the Invention]

Hereinafter, preferred embodiments of the present invention will be described in detail with reference to the accompanying drawings. Advantages and features of the present invention, and methods of achieving them, will become clear with reference to the embodiments described below in detail in conjunction with the accompanying drawings. However, the present invention is not limited to the embodiments disclosed below and may be implemented in various different forms, only the present embodiments make the disclosure of the present invention complete, it is provided to completely inform those skilled in the art of the scope of the invention to which the present invention belongs, and the present invention is only defined by the scope of the claims. Same reference numbers designate same components throughout the specification.

Unless otherwise defined, all terms (including technical and scientific terms) used in this specification may be used in a meaning commonly understood by those of ordinary skill in the art to which the present invention belongs. In addition, terms defined in commonly used dictionaries are not interpreted ideally or excessively unless explicitly specifically defined.

In addition, the terms used in the present specification are for describing the embodiments and are not intended to limit the present invention. The terms "comprises" and/or "comprising" as used in the specification do not exclude the presence or addition of one or more other components, steps and/or operations in addition to the components, steps and/or operations mentioned. And "and/or" includes each and every combination of one or more of the mentioned items.

In addition, terms such as first, second, A, B, (a), and (b) may be used in describing components of an embodiment of the present invention. These terms are only used to distinguish the component from other components, and the nature, order, or order of the corresponding component is not limited by the term. When a component is described as being 'connected', 'coupled' or 'interconnected' to another component, the component may be directly connected, coupled or interconnected to the other component, but not between the component and the other component, but it should be understood that another component may be 'connected', 'coupled' or 'interconnected' between the component and the other component.

An 'optical axis direction' used below is defined as an optical axis direction of a lens coupled to a lens driving device. Meanwhile, the 'optical axis direction' may correspond to 'up and down directions' and 'z-axis directions'.

Hereinafter, the present embodiment will be described in more detail according to the accompanying drawings.

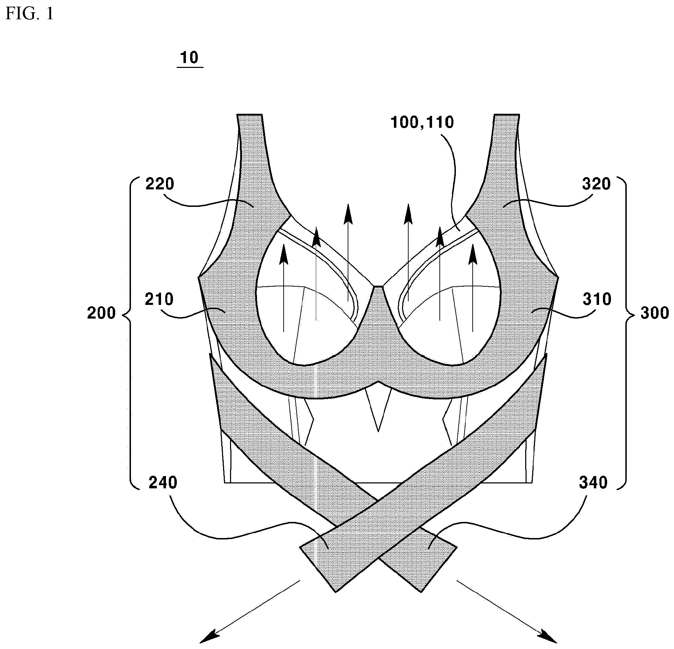

FIG. 1 is a front view of a female top for breast correction and thoracic spine correction according to present invention.

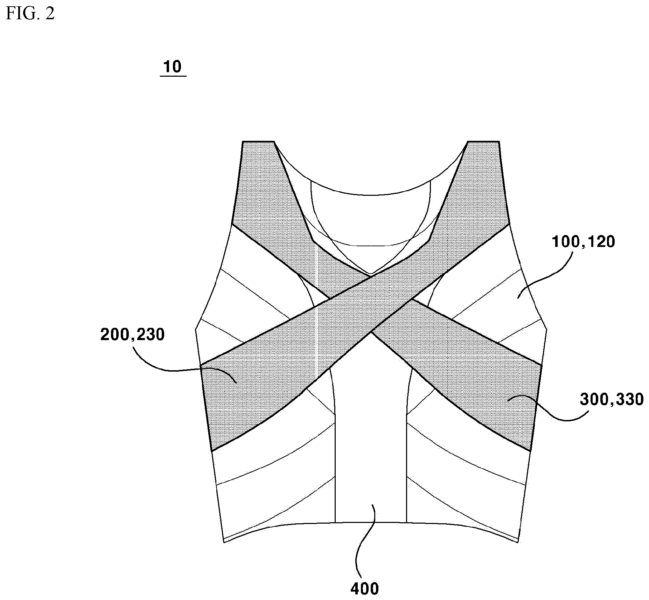

FIG. 2 is a rear view of a female top for breast correction and thoracic spine correction according to the present invention.

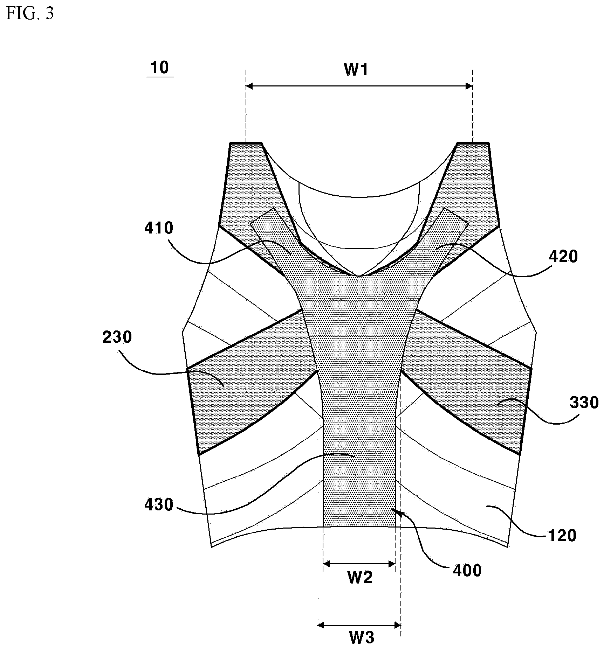

FIG. 3 is a rear view of a female top for breast correction and thoracic spine correction according to the present invention.

Referring to Figures 1 to 4, women's top **10** for breast correction and thoracic spine correction according to the present invention includes a main body **100,** a first band **200,** a second band **300,** and a lumbar vertebra support **400.**

The women's top **10** for breast correction and thoracic spine correction includes a main body **100.** The main body **100** covers the user's upper body. The main body **100** may surround the user's shoulder, breast, and back. At least a portion of the main body **100** may be formed as a corset surface. The body **100** may include a wire. The main body **100** may be formed of an elastic material. The main body **100** comprises a front surface **110** and a rear surface **120.** The front surface **110** may be a direction directing forward when the user wears the women's top **10** for breast correction and thoracic spine correction. The rear surface **120** may be a direction directing rearward when the user wears the women's top **10** for breast correction and thoracic spine correction. The rear surface **120** may be disposed at an opposite side of the front surface **110.** The front surface **120** and the rear surface **120** may be integrally formed.

The women's top **10** for breast correction and thoracic spine correction includes a first band **200.** The first band **200** is disposed in the main body **100.** The first band **200** may be disposed on the front surface **110** of the main body **100.** The first band **200** may be disposed on the rear surface **120** of the main body **100.**

The first band **200** may be formed of only an elastic material. The first band **200** may be formed only of inelastic material. The first band **200** may be formed of an elastic material and an inelastic material. Specifically, a portion of the first band **200** is formed of an elastic material, and the remaining portion may be formed of an inelastic material.

The first band **200** includes a first round part **210.** The first round part **210** is disposed on the front surface **110** of the main body **100.** One end portion of the first round part **210** is coupled with one end portion of a second round part **310,** which will be described later. The first round part **210** is placed below the user's breast when the user wears the women's top **10** for breast correction and thoracic spine correction. The user's breast of the first round part **210** can be raised upward from below.

The first band **200** may include a first portion **220.** The first portion **220** of the first band **200** extends upward from the other end portion of the first round part **210.**

The first band **200** includes a second portion **230.** The second portion **230** of the first band **200** extends from the first portion **220** of the first band **200** to the rear surface **120** of the main body **100.** The second portion **230** of the first band **200** may be intersected with a fifth portion **330** of the second band **300,** which will be described later, at the rear surface **120** of the main body **100.** At this time, the second portion **230** of the first band **200** may be intersected with the fifth portion **330** of the second band **300** in the shape of a letter 'X'. At least a portion of the second portion **230** of the first band **200** may be overlapped with the fifth portion **330** of the second band **300.**

The first band **200** may include a third portion **240.** The third portion **240** of the first band **200** may be extended from the second portion **230** of the first band **200** to the front surface **110** of the main body **100.** The third portion **240** of the first band **200** is disposed below the first round part **210** and below a second round part **310,** which will be described later. The third portion **240** of the first band **200** is coupled with a sixth portion **340** of the second band **300,** which will be described later. At this time, the first and second bands **200** and **300** may be fixed to the main body **100.**

The women's top **10** for breast correction and thoracic spine correction includes a second band **300.** The second band **300** is disposed in the main body **100.** The second band **300** may be disposed on the front surface **110** of the main body **100.** The second band **300** may be disposed on the rear surface **120** of the main body **100.** The second band **300** is coupled with the first band **200.**

The second band **300** may be formed only of an elastic material. The second band **300** may be formed only of an inelastic material. The second band **300** may be formed of an elastic material and an inelastic material. Specifically, a portion of the second band **300** may be formed of an elastic material, and the remaining portion may be formed of an inelastic material.

The second band **300** includes a second round part **310.** The second round part **310** is disposed on the front surface **110** of the main body **100.** One end portion of the second round part **310** is coupled with one end portion of the first round part **210.** The second round part **310** is disposed below the user's breast when the user wears the women's top **10** for breast correction and thoracic spine correction. The user's breast of the second round part **310** can be raised upward from below.

The second band **300** includes a fourth portion **320.** The fourth portion **320** of the second band **300** being extended upward from the other end portion of the second round part **310.**

The second band **300** includes a fifth portion **330.** The fifth portion **330** of the second band **300** may be extended from the fourth portion **320** of the second band **300** to the rear surface **120** of the main body **100.** The fifth portion **330** of the second band **300** being intersected with the second portion **230** of the first band **200** at the rear surface **120** of the main body **100.** At this time, the fifth portion **330** of the second band **300** may be intersected with the second portion **230** of the first band **200** in the shape of a letter 'X'. At least a portion of the fifth portion **330** of the second band **300** may be overlapped with the second portion **230** of the first band **200.**

The second band **300** includes a sixth portion **340.** The sixth portion **340** of the second band **300** being extended from the fifth portion **330** of the second band **300** to the front surface **110** of the main body **100.** The sixth portion **340** of the second band **300** is disposed below the second round part **310** and below the first round part **210.** The sixth portion **340** of the second band **300** is coupled with the third portion **240** of the first band **200.** At this time, the first and second bands **200** and **300** may be fixed to the main body **100.**

The third portion **240** of the first band **200** is pulled in a first direction. The sixth portion **340** of the second band **300** is pulled in a second direction. The first direction is a direction intersecting the second direction. In this case, the intersection angle of the first direction and the second direction is an obtuse angle. The intersection angle between the first direction and the second direction may be 120 degrees.

When the third portion **240** of the first band **200** is pulled in a first direction and the sixth portion **340** of the second band **300** is pulled in a second direction, the first round part **210** is pulled in a third direction. The third direction is a direction being intersected with the first direction and the second direction. The intersection angle between the third direction and the first direction is an obtuse angle. The intersection angle between the third direction and the second direction is an obtuse angle. An intersection angle being formed by each of the first to third directions may be 120 degrees. Through this, the force pulling the third portion **240** of the first band **200** and the sixth portion **340** of the second band **300** can be evenly distributed. In this case, the tensile force may be evenly distributed throughout the first and second bands **200** and **300.** The third direction may be an upward direction of the first and second round parts **210** and **310.** Through this, the breast can be pulled upward so that it does not sag.

The third portion **240** of the first band **200** and the sixth portion **340** of the second band **300** are coupled to each other at the front surface **110** of the main body **100.** As a coupling method, there may be a ribbon type, a strap type, a hook type, a belt type, a Velcro type, a button type, and the like.

The third portion **240** of the first band **200** and the sixth portion **340** of the second band **300** may be coupled in a ribbon type. The third portion **240** of the first band **200** and the sixth portion **340** of the second band **300** can be coupled by being tied in various shapes such as ribbons and ties on the front surface **110** of the main body **100.** At this time, the user can adjust the tying strength to adjust the correction effect according to the user's body. Through this, the first and second round parts **210** and **310** can lift the weight of the breast from below the breast by lever principle. At the same time, the lumbar vertebra support **400,** which will be described later, is pressurized to maintain a correct posture.

The third portion **240** of the first band **200** and the sixth portion **340** of the second band **300** may be coupled in a strap type. The third portion **240** of the first band **200** may include a length adjusting member. The length adjusting member may include a hole. The length adjusting member may include first and second holes being spaced apart from each other. The second hole of the length adjusting member may be disposed below the first hole of the length adjusting member. A first portion **240** of the first band **200** may penetrate through and be fixed to the first hole of the length adjusting member. The sixth portion **340** of the second band **300** may penetrate through the first hole of the length adjusting member of the third portion **240** of the first band **200.** The sixth portion **340** of the second band **300** penetrating through the first hole of the length adjusting member may penetrate through the second hole of the length adjusting member. In this case, the size of the sixth portion **340** of the second band **300** may be adjustable by penetrating through the first and second holes of the length adjusting member. Through this, the lengths of the first and second bands **200** and **300** can be adjusted to be suitable to the user's body characteristics. In addition, the lengths of the first and second bands **200** and **300** may be adjusted according to areas or strengths requiring correction. Through this, the first and second round parts **210** and **310** can lift the weight of the breast below the breast by lever principle. At the same time, a lumbar vertebra support **400,** which will be described later, is pressurized to maintain a correct posture.

The third portion **240** of the first band **200** and the sixth portion **340** of the second band **300** may be coupled in a hook type. Specifically, the third portion **240** of the first band **200** may include a ring in which a hole is formed. The sixth portion **340** of the second band **300** may include a hook. The hook of the sixth portion **340** of the second band **300** may be coupled to the hook of the third portion **240** of the first band **200.** However, it is not limited thereto, and the third portion **240** of the first band **200** may include a hook, and the sixth portion **340** of the second band **300** may include a hole. The number of holes in the third portion **240** of the first band **200** may be plural. The hooks of the sixth portion **340** of the second band **300** may be plural. Through this, it is possible to adjust the user's body size, the user's body characteristics, the body part to be corrected, and the intensity of correction. Through this, the first and second round parts **210** and **310** can lift the weight of the breast below the breast by lever principle. At the same time, the lumbar vertebra support **400,** which will be described later, is pressurized to maintain a correct posture.

The third portion **240** of the first band **200** and the sixth portion **340** of the second band **300** may be coupled in a belt type. Specifically, the third portion **240** of the first band **200** may include a buckle. The buckle may include a pin. A plurality of holes may be formed in the sixth portion **340** of the second band **300.** The sixth portion **340** of the second band **300** may penetrate through the buckle of the third portion **240** of the first band **200.** At this time, the pin of the buckle may be inserted into and fixed to any one of the plurality of holes of the sixth portion **340** of the second band **300.** In this case, the pin of the buckle can be inserted into the hole by adjusting the length of the sixth portion **340** of the second band **300** in consideration of the user's body characteristics, body size, desired region to be corrected, and intensity of correction. However, it is not limited thereto, and a plurality of holes are formed in the third portion **240** of the first band **200,** and the sixth portion **340** of the second band **300** may include a buckle.

The third portion **240** of the first band **200** and the sixth portion **340** of the second band **300** may be coupled in a Velcro type. Specifically, the third portion **240** of the first band **200** may include a first adhesive member. The sixth portion **340** of the second band **300** may include a second adhesive member. Through this, the first adhesive member and the second adhesive member may be coupled to each other. The first adhesive member may be disposed on the entire third portion **240** of the first band **200.** The second adhesive member may be disposed on the entire sixth portion **340** of the second band **300.** Through this, the length and size of the first and second bands **200** and **300** can be adjusted in consideration of the user's body characteristics, body size, region to be corrected, and intensity of correction. Through this, the first and second round parts **210** and **310** can lift the weight of the breast below the breast by lever principle. At the same time, the lumbar vertebra support **400,** which will be described later, is pressurized to maintain a correct posture.

The third portion **240** of the first band **200** and the sixth portion **340** of the second band **300** may be coupled in a button type. At this time, the button may mean a snap button or a click button. Specifically, a female button for a snap button is disposed in the third portion **240** of the first band **200,** and a male button may be disposed in the sixth portion **340** of the second band **300.** In addition, a male button for a snap button may be disposed in the third portion **240** of the first band **200,** and a female button for a snap button may be disposed in the sixth portion **340** of the second band **300.** A plurality of female buttons for snap buttons and male buttons for snap buttons may be provided. Through this, it is possible to adjust the length of the first and second bands **200** and **300** according to the user's body characteristics, size, and body part to be corrected. The first band **200** and the second band **300** may be fixed as the female button for snap button and the male button for snap button are coupled to each other.

The third portion **240** of the first band **200** and the sixth portion **340** of the second band **300** are extended from the rear surface **120** of the main body **100** to the front surface **100,** thereby surrounding the abdominal area. In this case, the lengths of the third portions **240** and **340** in a vertical direction may be increased. In this case, upper ends of the second portions **240** and **340** may be overlapped with lower ends of the first and second round parts **210** and **310.**

The women's top **10** for breast correction and thoracic spine correction includes a lumbar vertebra support **400.** The lumbar vertebra support **400** is disposed on the main body **100.** The lumbar vertebra support **400** is disposed on the rear surface **120** of the main body **100.** The lumbar vertebra support **400** may be disposed between the first and second bands **200** and **300** and the main body **100.** Or, the lumbar vertebra support **400** may be disposed between the outer and inner surfaces of the rear surface **120** of the main body **100.** FIG. 3 is a drawing illustrating the lumbar vertebra support **400** at an outer side of the first and second bands **200** and **300** to confirm the arrangement relationship between the lumbar vertebra support **400** and the first and second bands **200** and **300.**

The lumbar vertebra support **400** includes a first section **410.** The first section **410** being overlapped with the first band **200.** The first section **410** may be overlapped with at least a portion of the second portion **230** of the first band **200.** The lumbar vertebra support **400** includes a second section **420.** The second section **420** being overlapped with the second band **300.** The second section **420** may be overlapped with at least a portion of the fifth portion **330** of the second band **200.** The lumbar vertebra support **400** includes a third section **430.** The third section **430** being extended downward from the first and second sections **410** and **420.**

The lumbar vertebra support **400** may be formed of a silicon material. However, it is not limited thereto, and may be formed of an elastic material. The lumbar vertebra support **400** may be pressed in a direction directing from the rear surface **120** of the main body **100** to the front surface **110** of the main body **100** by the first and second bands **200** and **300.** The lumbar vertebra support **400** may be deformed by the first and second bands **200** and **300.** The degree of deformation of the first and second sections **410** and **420** of the lumbar vertebra support **400** may be greater than the degree of deformation of the third section **430** of the lumbar vertebra support **400.** Through this, the user's spine, back, and shoulders can be stretched.

The lumbar vertebra support **400** may be formed in the shape of a letter 'Y'. The lumbar vertebra support **400** may be disposed at the central portion of the main body **100.** The lumbar vertebra support **400** may be formed symmetrically about the central portion of the main body **100.** The lumbar vertebra support **400** may have a narrower width in the horizontal direction as it travels downward from above. Specifically, the maximum width **W1** in the horizontal direction between the first section **410** and the second section **420** of the lumbar vertebra support **400** may be formed to be larger than the width **W2** in the corresponding direction of the third section **430** of the lumbar vertebra support **400.** The shortest width **W3** between the first section **410** and the second section **420** of the lumbar vertebra support **400** in the horizontal direction may be formed to be larger than the width **W2** in the corresponding direction of the third section **430** of the lumbar vertebra support **400.** Through this, the user's thoracic vertebrae and cervical vertebrae may be intensively pressed to induce a correct posture.

Although the embodiment of the present invention has been described above with reference to the accompanying drawings, those of ordinary skill in the art to which the present invention belongs will understand that the present invention is defined in the appended set of claims.

### [Description of the Symbols]

| | | | |
|---|---|---|---|
| 100: | main body | 200: | first band |
| 300: | second band | 400: | lumbar vertebra support |

## Claims

1. A women's top for breast shaping and thoracic spine correction comprising:
a main body (100) surrounding the upper body of a user;
a first band (200) being disposed on one side of the main body (100);
a second band (300) being disposed on the other side of the main body (100) and at least a portion of which is connected, on a front surface of the main body (100), to the first band (200); and
a lumbar vertebra support (400) being disposed on a rear surface of the main body (100),
wherein the lumbar vertebra support (400) includes a first section (410) being overlapped with the first band (200), a second section (420) being overlapped with the second band (300), and a third section (430) being extended downward from the first and second sections (410) and (420),
wherein the first band (200) includes a first round part (210) being disposed on a front surface of the main body (100) and disposed below one breast of a user,
wherein the second band (300) includes a second round part (310) being disposed on a front surface of the main body (100) and disposed below the user's other breast,
wherein one end of the first round part (210) and one end of the second round part (310) are interconnected,
wherein the first band (200) includes a first portion (220) being extended upward from the other end of the first round part (210),
wherein the second band (300) includes a fourth portion (320) being extended upward from the other end of the second round part (310),
wherein the first band (200) includes a second portion (230) being extended from the first portion (220) to a rear surface of the main body (100),
wherein the second band (300) includes a fifth portion (330) being extended from the fourth portion (320) to a rear surface of the main body (100),
wherein the second portion (230) and the fifth portion (330) being intersected at a rear surface of the main body (100),
**characterized in that**,
the first band (200) includes a third portion (240) being extended again from the second portion (230) to a front surface of the main body (100),
wherein the second band (300) includes a sixth portion (340) being extended again from the fifth portion (330) to a front surface of the main body (100),
wherein the third portion (240) and the sixth portion (340) are disposed below the first round part (210) and the second round part (310),
wherein the third portion (240) and the sixth portion (340) are coupled to each other and fixed at a front surface of the main body (100),
wherein in use the third portion (240) is pulled in a first direction,
wherein the sixth portion (340) is pulled in a second direction being intersected by the first direction,
wherein the first round part (210) and the second round part (310) are pulled in a third direction when the third portion (240) is pulled in the first direction and the sixth portion (340) is pulled in the second direction, and
wherein the intersection angle of the first direction and the second direction is an obtuse angle, wherein the intersection angle of the second direction and the third direction is an obtuse angle, and wherein the intersection angle of the first direction and the third direction is an obtuse angle.

2. The women's top for breast shaping and thoracic spine correction according to claim 1,
wherein the intersection angle of the first direction and the second direction, the intersection angle of the second direction and the third direction, and the intersection angle of the first direction and the third direction are 120 degrees respectively.

3. The women's top for breast shaping and thoracic spine correction according to claim 1,
wherein the third direction is a direction directing upward from the first and second round parts (210) and (310).

4. The women's top for breast shaping and thoracic spine correction according to claim 1,
wherein the first section (410) of the lumbar vertebra support (400) is overlapped with at least a portion of the second portion (230) of the first band (200), and
wherein the second section (420) of the lumbar vertebra support (400) is overlapped with at least a portion of the fifth portion (330) of the second band (300).

5. The women's top for breast shaping and thoracic spine correction according to claim 1,
wherein the lumbar vertebra support (400) is pressed in a direction directing from a rear surface of the main body (100) to a front surface of the main body (100) by the first and second bands (200) and (300).

6. The women's top for breast shaping and thoracic spine correction according to claim 5,
wherein the lumbar vertebra support (400) is formed of a silicon material, and
wherein the lumbar vertebra support (400) is deformed by the deformation of the first and second bands (200) and (300).

7. The women's top for breast shaping and thoracic spine correction according to claim 6,
wherein the degree to which the first and second sections (410) and (420) of the lumbar vertebra support (400) are deformed is greater than the degree to which the third section (430) of the lumbar vertebra support (400) is deformed.

8. The women's top for breast shaping and thoracic spine correction according to claim 7,
wherein the maximum horizontal distance between the first section (410) and the second section (420) of the lumbar vertebra support (400) is greater than the width of the third section (430) of the lumbar vertebra support (400).

9. The women's top for breast shaping and thoracic spine correction according to claim 8,
wherein the shortest distance in the horizontal direction between the first section (410) and the second section (420) of the lumbar vertebra support (400) is greater than the width in the corresponding direction of the third section (430) of the lumbar vertebra support (400).

## Patentansprüche

1. Oberteil für Frauen zur Brustformung und Korrektur der Brustwirbelsäule, umfassend:
einen Hauptkörper (100), der den Oberkörper einer Benutzerin umgibt;
ein erstes Band (200), das auf einer Seite des Hauptkörpers (100) angeordnet ist;
ein zweites Band (300), das auf der anderen Seite des Hauptkörpers (100) angeordnet ist und von dem mindestens ein Abschnitt auf einer Vorderseite des Hauptkörpers (100) mit dem ersten Band (200) verbunden ist; und
eine Lendenwirbel-Stütze (400), die auf einer Rückseite des Hauptkörpers (100) angeordnet ist,
wobei die Lendenwirbel-Stütze (400) einen ersten Abschnitt (410) umfasst, der mit dem ersten Band (200) überlappt, einen zweiten Abschnitt (420), der mit dem zweiten Band (300) überlappt, und einen dritten Abschnitt (430), der sich von den ersten und zweiten Abschnitten (410) und (420) nach unten erstreckt,
wobei das erste Band (200) einen ersten rundförmigen Abschnitt (210) umfasst, der auf einer Vorderseite des Hauptkörpers (100) und unterhalb einer Brust der Benutzerin angeordnet ist,
wobei das zweite Band (300) einen zweiten rundförmigen Abschnitt (310) umfasst, der auf einer Vorderseite des Hauptkörpers (100) und unterhalb der anderen Brust der Benutzerin angeordnet ist,
wobei ein Ende des ersten rundförmigen Abschnitts (210) und ein Ende des zweiten rundförmigen Abschnitts (310) miteinander verbunden sind,
wobei das erste Band (200) einen ersten Abschnitt (220) umfasst, der sich von dem anderen Ende des ersten rundförmigen Abschnitts (210) nach oben erstreckt,
wobei das zweite Band (300) einen vierten Abschnitt (320) umfasst, der sich von dem anderen Ende des zweiten rundförmigen Abschnitts (310) nach oben erstreckt,
wobei das erste Band (200) einen zweiten Abschnitt (230) umfasst, der sich vom ersten Abschnitt (220) zu einer Rückseite des Hauptkörpers (100) erstreckt,
wobei das zweite Band (300) einen fünften Abschnitt (330) umfasst, der sich vom vierten Abschnitt (320) zu einer Rückseite des Hauptkörpers (100) erstreckt,
wobei sich der zweite Abschnitt (230) und der fünfte Abschnitt (330) auf einer Rückseite des Hauptkörpers (100) kreuzen,
**dadurch gekennzeichnet, dass** das erste Band (200) einen dritten Abschnitt (240) umfasst, der sich erneut vom zweiten Abschnitt (230) zu einer Vorderseite des Hauptkörpers (100) erstreckt,
wobei das zweite Band (300) einen sechsten Abschnitt (340) umfasst, der sich erneut vom fünften Abschnitt (330) zu einer Vorderseite des Hauptkörpers (100) erstreckt,
wobei der dritte Abschnitt (240) und der sechste Abschnitt (340) unterhalb des ersten rundförmigen Abschnitts (210) und des zweiten rundförmigen Abschnitts (310) angeordnet sind,
wobei der dritte Abschnitt (240) und der sechste Abschnitt (340) miteinander gekoppelt sind und an einer Vorderseite des Hauptkörpers (100) befestigt sind, wobei im Gebrauch der dritte Abschnitt (240) in eine erste Richtung gezogen wird, wobei der sechste Abschnitt (340) in eine zweite Richtung gezogen wird, die die erste Richtung schneidet,
wobei der erste rundförmige Abschnitt (210) und der zweite rundförmige Abschnitt (310) in eine dritte Richtung gezogen werden, wenn der dritte Abschnitt (240) in die erste Richtung und der sechste Abschnitt (340) in die zweite Richtung gezogen werden, und
wobei der Schnittwinkel zwischen der ersten Richtung und der zweiten Richtung ein stumpfer Winkel ist, wobei der Schnittwinkel zwischen der zweiten Richtung und der dritten Richtung ein stumpfer Winkel ist und wobei der Schnittwinkel zwischen der ersten Richtung und der dritten Richtung ein stumpfer Winkel ist.

2. Das Oberteil für Frauen zur Brustformung und Korrektur der Brustwirbelsäule nach Anspruch 1, wobei der Schnittwinkel zwischen der ersten Richtung und der zweiten Richtung, der Schnittwinkel zwischen der zweiten Richtung und der dritten Richtung und der Schnittwinkel zwischen der ersten Richtung und der dritten Richtung jeweils 120 Grad beträgt.

3. Das Oberteil für Frauen zur Brustformung und Korrektur der Brustwirbelsäule nach Anspruch 1, wobei die dritte Richtung eine Richtung ist, die sich von den ersten und zweiten rundförmigen Abschnitten (210) und (310) nach oben erstreckt.

4. Das Oberteil für Frauen zur Brustformung und Korrektur der Brustwirbelsäule nach Anspruch 1, wobei der erste Abschnitt (410) der Lendenwirbel-Stütze (400) mindestens einen Teil des zweiten Abschnitts (230) des ersten Bandes (200) überlappt, und wobei der zweite Abschnitt (420) der Lendenwirbel-Stütze (400) mindestens einen Teil des fünften Abschnitts (330) des zweiten Bandes (300) überlappt.

5. Das Oberteil für Frauen zur Brustformung und Korrektur der Brustwirbelsäule nach Anspruch 1, wobei die Lendenwirbel-Stütze (400) durch die ersten und zweiten Bänder (200) und (300) in eine Richtung von der Rückseite des Hauptkörpers (100) zur Vorderseite des Hauptkörpers (100) gepresst wird.

6. Das Oberteil für Frauen zur Brustformung und Korrektur der Brustwirbelsäule nach Anspruch 5, wobei die Lendenwirbel-Stütze (400) aus einem Silikonmaterial gebildet ist und wobei die Lendenwirbel-Stütze (400) durch die Deformation der ersten und zweiten Bänder (200) und (300) verformt wird.

7. Das Oberteil für Frauen zur Brustformung und Korrektur der Brustwirbelsäule nach Anspruch 6, wobei der Verformungsgrad der ersten und zweiten Abschnitte (410) und (420) der Lendenwirbel-Stütze (400) größer ist als der Verformungsgrad des dritten Abschnitts (430) der Lendenwirbel-Stütze (400).

8. Das Oberteil für Frauen zur Brustformung und Korrektur der Brustwirbelsäule nach Anspruch 7, wobei der maximale horizontale Abstand zwischen dem ersten Abschnitt (410) und dem zweiten Abschnitt (420) der Lendenwirbel-Stütze (400) größer ist als die Breite des dritten Abschnitts (430) der Lendenwirbel-Stütze (400).

9. Das Oberteil für Frauen zur Brustformung und Korrektur der Brustwirbelsäule nach Anspruch 8, wobei der kürzeste Abstand in horizontaler Richtung zwischen dem ersten Abschnitt (410) und dem zweiten Abschnitt (420) der Lendenwirbel-Stütze (400) größer ist als die Breite in der entsprechenden Richtung des dritten Abschnitts (430) der Lendenwirbel-Stütze (400).

## Revendications

1. Un haut pour femme destiné au modelage des seins et à la correction de la colonne thoracique, comprenant :
un corps principal (100) entourant le haut du corps d'une utilisatrice ;
une première bande (200) disposée sur un côté du corps principal (100) ;
une deuxième bande (300) disposée sur l'autre côté du corps principal (100) et dont au moins une partie est reliée, sur une surface avant du corps principal (100), à la première bande (200) ; et
un support de vertèbres lombaires (400) disposé sur une surface arrière du corps principal (100),
le support de vertèbres lombaires (400) comprenant une première section (410) chevauchant la première bande (200), une deuxième section (420) chevauchant la deuxième bande (300), et une troisième section (430) s'étendant vers le bas à partir des première et deuxième sections (410) et (420),
la première bande (200) comprenant une première partie arrondie (210) disposée sur une surface avant du corps principal (100) et située au-dessous d'un sein de l'utilisatrice,
la deuxième bande (300) comprenant une deuxième partie arrondie (310) disposée sur une surface avant du corps principal (100) et située au-dessous de l'autre sein de l'utilisatrice,
une extrémité de la première partie arrondie (210) et une extrémité de la deuxième partie arrondie (310) étant interconnectées,
la première bande (200) comprenant une première portion (220) s'étendant vers le haut à partir de l'autre extrémité de la première partie arrondie (210),
la deuxième bande (300) comprenant une quatrième portion (320) s'étendant vers le haut à partir de l'autre extrémité de la deuxième partie arrondie (310),
la première bande (200) comprenant une deuxième portion (230) s'étendant de la première portion (220) vers une surface arrière du corps principal (100),
la deuxième bande (300) comprenant une cinquième portion (330) s'étendant de la quatrième portion (320) vers une surface arrière du corps principal (100),
la deuxième portion (230) et la cinquième portion (330) se croisant sur une surface arrière du corps principal (100),
**caractérisé en ce que** la première bande (200) comprend une troisième portion (240) s'étendant de nouveau de la deuxième portion (230) vers une surface avant du corps principal (100),
la deuxième bande (300) comprenant une sixième portion (340) s'étendant de nouveau de la cinquième portion (330) vers une surface avant du corps principal (100),
la troisième portion (240) et la sixième portion (340) étant disposées au-dessous de la première partie arrondie (210) et de la deuxième partie arrondie (310),
la troisième portion (240) et la sixième portion (340) étant accouplées l'une à l'autre et fixées sur une surface avant du corps principal (100),
en usage, la troisième portion (240) étant tirée dans une première direction,
la sixième portion (340) étant tirée dans une deuxième direction qui coupe la première direction,
la première partie arrondie (210) et la deuxième partie arrondie (310) étant tirées dans une troisième direction lorsque la troisième portion (240) est tirée dans la première direction et que la sixième portion (340) est tirée dans la deuxième direction, et
l'angle d'intersection entre la première direction et la deuxième direction étant obtus, l'angle d'intersection entre la deuxième direction et la troisième direction étant obtus, et l'angle d'intersection entre la première direction et la troisième direction étant obtus.

2. Le haut pour femme selon la revendication 1, dans lequel l'angle d'intersection entre la première direction et la deuxième direction, l'angle d'intersection entre la deuxième direction et la troisième direction, et l'angle d'intersection entre la première direction et la troisième direction sont respectivement de 120 degrés.

3. Le haut pour femme selon la revendication 1, dans lequel la troisième direction est une direction se dirigeant vers le haut à partir des première et deuxième parties arrondies (210) et (310).

4. Le haut pour femme selon la revendication 1, dans lequel la première section (410) du support de vertèbres lombaires (400) chevauche au moins une partie de la deuxième portion (230) de la première bande (200), et dans lequel la deuxième section (420) du support de vertèbres lombaires (400) chevauche au moins une partie de la cinquième portion (330) de la deuxième bande (300).

5. Le haut pour femme selon la revendication 1, dans lequel le support de vertèbres lombaires (400) est pressé dans une direction allant d'une surface arrière du corps principal (100) vers une surface avant du corps principal (100) par les première et deuxième bandes (200) et (300).

6. Le haut pour femme selon la revendication 5, dans lequel le support de vertèbres lombaires (400) est constitué d'un matériau en silicone, et dans lequel le support de vertèbres lombaires (400) est déformé par la déformation des première et deuxième bandes (200) et (300).

7. Le haut pour femme selon la revendication 6, dans lequel le degré de déformation des première et deuxième sections (410) et (420) du support de vertèbres lombaires (400) est supérieur au degré de déformation de la troisième section (430) du support de vertèbres lombaires (400).

8. Le haut pour femme selon la revendication 7, dans lequel la distance horizontale maximale entre la première section (410) et la deuxième section (420) du support de vertèbres lombaires (400) est supérieure à la largeur de la troisième section (430) du support de vertèbres lombaires (400).

9. Le haut pour femme selon la revendication 8, dans lequel la distance horizontale la plus courte entre la première section (410) et la deuxième section (420) du support de vertèbres lombaires (400) est supérieure à la largeur, dans la direction correspondante, de la troisième section (430) du support de vertèbres lombaires (400).
